(19) **Europäisches Patentamt / European Patent Office / Office européen des brevets**

(11) **EP 2 146 688 B1**

(12) **EUROPEAN PATENT SPECIFICATION**

(45) Date of publication and mention
of the grant of the patent:
**18.03.2015 Bulletin 2015/12**

(21) Application number: **07834405.8**

(22) Date of filing: **30.11.2007**

(51) Int Cl.:
*A61K 8/97* <sup>(2006.01)</sup>     *A61Q 19/02* <sup>(2006.01)</sup>

(86) International application number:
**PCT/KR2007/006123**

(87) International publication number:
**WO 2009/069839 (04.06.2009 Gazette 2009/23)**

(54) **Use of an extract of magnolia sieboldii for skin whitening**

Verwendung von einem Extrakt aus Magnolia sieboldii zur Aufhellung der Haut

Utlisation d'un extrait de Magnolia sieboldii pour blanchir la peau

(84) Designated Contracting States:
**AT BE BG CH CY CZ DE DK EE ES FI FR GB GR HU IE IS IT LI LT LU LV MC MT NL PL PT RO SE SI SK TR**

(43) Date of publication of application:
**27.01.2010 Bulletin 2010/04**

(73) Proprietor: **Coreana Cosmetics Co., Ltd.**
**Chungcheongnam-do**
**Cheonan-shi 330-833 (KR)**

(72) Inventors:
• **LEE, Ghang Tai**
**Cheonan-Si**
**Chungcheongnam-do 330-835 (KR)**
• **LEE, Jung Noh**
**Chungcheongnam-do 339-752 (KR)**
• **LEE, Song Yi**
**Gyeonggi-do 440-710 (KR)**
• **LEE, Seung Ji**
**Chungcheongnam-do 330-786 (KR)**
• **LEE, Kun Kook**
**Seoul 138-788 (KR)**

(74) Representative: **Catherine, Alain et al**
**Cabinet HARLE et PHELIP**
**14-16, rue Ballu**
**75009 Paris (FR)**

(56) References cited:
**CN-A- 1 493 271        JP-A- 9 077 635**
**KR-A- 19980 025 843**

• **DATABASE WPI Week 200042 Thomson Scientific, London, GB; AN 2000-476612 XP002715856, & CN 1 244 572 A (ZHANG G) 16 February 2000 (2000-02-16)**
• **NA, M. K. ET AL.: 'Inhibitory Activity of Medicinal Plant Extracts against Tyrosinase' KOREAN JOURNAL OF KOREAN MEDICAL INSTITUTE OF DERMATOLOGY & AESTHETICS vol. 1, no. 1, 2005, pages 91 - 97**

Remarks:
The file contains technical information submitted after the application was filed and not included in this specification

## Description

[Technical Field]

The present invention relates to the use of a cosmetic composition comprising an extract or *Magnolia sieboldii* as an active ingredient for skin whitening.

[Background Art]

The pigment of human skin or hair functions to protect the skin or hair from the deleterious effects of sunlight, particularly UV light. Persons lacking this pigment are very sensitive to sunlight, so that they are more likely to get burned. Also, there is a high likelihood of occurrence of skin cancer, even at young ages. It is known that short-wavelength UV rays (290-320 nm) and carcinogenic substances form harmful radicals, for example, oxygen radicals, on the skin, and such oxygen radicals attack skin cells to cause skin aging. The main function of melanin is to scavenge such harmful radicals, thus protecting the skin from damage caused by such harmful radicals. Accordingly, people having plenty of melanin have an effective defense system capable of protecting the skin from physical or chemical toxic substances. Factors promoting the production of melanin include, in addition to sunlight (UV light), estrogens and prostaglandins. Melanin is produced at melanocytes by the conversion of tyrosine to dopa, dopachrome through the action of the enzyme tyrosinase, followed by complex oxidation and condensation reactions. The produced melanin are transferred into skin cells and lost with epidermal peeling. This melanin production process is a naturally occurring phenomenon, and in a normal human skin, the overproduction of melanin does not occur. However, when the skin responds to external stimuli, for example, UV light, environmental pollutants or stresses, the overproduction of melanin will occur. When the overproduced melanin remains in the skin without being discharged out of the skin, pigmentation will occur. Among the above-described external stimuli, UV light is the greatest source stimulating melanin biosynthesis and can influence various processes associated with melanin production. That is, UV light acts as an important factor which induces the overproduction of melanin by promoting the activity of melanocytes, the secretion of hormones stimulating melanin biosynthesis, the oxidation of melanin, or the activity of tyrosinase.

The greatest characteristic of this melanin production mechanism is that only one enzyme, tyrosinase, is involved in the melanin production mechanism. When the tyrosinase activity is inhibited to prevent the production of melanin, a skin-whitening effect can be expected.

In the prior art, it is known to use ascorbic acid as a whitening substance (Japanese Patent Laid-Open Publication No. Hei 4-9320). However, ascorbic acid has a problem in that the activity thereof decreases with the passage of time, because the phase stability thereof in a formulation is poor. For this reason, various methods of stabilizing ascorbic acid with capsules or liposomes have recently been suggested, but a reliable stabilization method has not yet been suggested.

Also, it is known to use hydroquinone as another whitening substance (Japanese Patent Laid-Open Publication No. Hei 6-192062). Hydroquinone has excellent effects, but the use thereof in cosmetic products has been limited, because it is a carcinogenic substance.

Moreover, it is known to use kojic acid as still another whitening substance (Japanese Patent Laid-Open Publication No. Sho 56-7710). Kojic acid shows an excellent whitening effect due to an excellent ability to inhibit tyrosinase, but it has a problem in terms of the stability thereof in a formulation and was recently reported to be a carcinogenic substance.

In addition, it is known to use arbutin as still another whitening substance (Japanese Patent Laid-Open Publication No. Hei 4-9315). Arbutin can be extracted from bearberries growing in alpine belts or can be obtained through synthesis. Also, it has a proven ability to inhibit tyrosinase, like kojic acid. However, arbutin has a structure in which sugar binds to hydroquinone, and it has a problem in that, when it is applied in cosmetic products, the sugar and the hydroquinone is separated by skin enzymes, such that the hydroquinone induces skin irritation.

Various patents and scientific articles are referred to and cited throughout the specification. The disclosure of the cited patents and scientific articles is incorporated herein by reference in its entirety, such that the general knowledge of the technical field to which the present invention pertains and the content of the present invention are more clearly explained.

[Disclosure]

[Technical Problem]

The present inventors have researched various skin physiological activities of natural plant components in order to improve skin pigmentation and, as a result, have found that, among natural plant extracts, an extract of *Magnolia sieboldii* has excellent effects of inhibiting tyrosinase activity and melanin production, causes no skin irritation and has a very excellent skin-whitening effect, thereby completing the present invention.

[Technical Solution]

Accordingly, it is an object of the present invention to provide a skin-whitening cosmetic composition comprising an extract of *Magnolia sieboldii* as an active ingredient.

[Advantageous Effects]

The present invention provides a skin-whitening cosmetic composition comprising an extract of *Magnolia sieboldii* as an active ingredient. The *Magnolia sieboldii* extract of the present invention has excellent effects of inhibiting tyrosinase activity and inhibiting the melanin synthesis of melanocytes, compared to those of the prior whitening components. Also, the inventive cosmetic composition comprising the *Magnolia sieboldii* extract as an active ingredient has a very excellent skin-whitening effect.

[Best Mode]

The present invention provides the use of a cosmetic composition comprising an extract of *Magnolia sieboldii* as an active ingredient for skin whitening.

*Magnolia sieboldii,* which is the active ingredient of the cosmetic composition of the present invention, is a deciduous small tree belonging to the *Magnoliaceae* family, and the *Magnolia sieboldii* extract is obtained from the flower of *Magnolia sieboldii.*

*Magnolia sieboldii* is about 7 meters in height, and the leaves thereof are alternate, long oval-shaped and glassy. The white large flowers thereof open downward in May and June, and the fruits thereof are ripe in September. It is an ornamental plant, grows in the valleys of mountains and is distributed in all parts of Korea except for Hamgyeongbuk-do, Japanese and China.

The *Magnolia sieboldii* extract that is used in the present invention can be obtained from the flower, leaf, branch and root of a *Magnolia sieboldii* tree, preferably the flower or branch of the tree, and more preferably the flower.

The *Magnolia sieboldii* extract of the present invention can be prepared according to any conventional method known in the art, under conditions of conventional temperature and pressure using a conventional solvent.

The *Magnolia sieboldii* extract that is the active ingredient of the inventive cosmetic composition is preferably obtained by extraction with an extraction solvent selected from the group consisting of water, an anhydrous or hydrous lower alcohol having 1-4 carbon atoms, acetone, ethyl acetate, butyl acetate and 1,3-butylene glycol, more preferably ethyl alcohol, butanol or methanol, and even more preferably, ethyl alcohol. Said ethyl alcohol is preferably 70% ethyl alcohol. The amount of the extraction solvent is preferably 1-10 times, more preferably 5-8 times, and most preferably 6 times the dry weight of *Magnolia sieboldii.*

The *Magnolia sieboldii* extract of the present invention is prepared through a process comprising the steps of:

(a) adding to dry *Magnolia sieboldii* an extraction solvent selected from the group consisting of water, an anhydrous or hydrous lower alcohol having 1-4 carbon atoms, acetone, ethyl acetate, butyl acetate and 1,3-butylene glycol as an amount of 1-10 times the drug weight of *Magnolia sieboldii*;

(b) extracting the solvent-added *Magnolia sieboldii* of step (a) by heating either at 40-100 °C for 3-20 hours or at 4-40 °C for 1-15 days;

(c) filtering the extract of step (b), aging the filtrate by allowing it to stand at 5-10 °C for 7-10 days, and then additionally filtering the aged filtrate; and

(d) drying the filtrate of step (c) in a rotary vacuum evaporator.

When 1,3-butylene glycol is used as the extraction solvent, the loss of dry weight is adjusted to 1 %(w/w) after carrying out the extraction step (b), because it is difficult to dry the filtrate using the rotary vacuum evaporator.

Meanwhile, the scope of the *Magnolia sieboldii* extract of the present invention includes not only the extract obtained through the above-described extraction method, but also an extract obtained through a conventional purification process. It is understood that fractions obtained through various additional purification methods, for example, separation with an ultrafiltration membrane having a given molecular weight cut-off, separation by various chromatography systems (manufactured for separation according to size, charge, hydrophobicity or affinity), are included in the scope of the *Magnolia sieboldii* extract of the present invention.

The *Magnolia sieboldii* extract of the present invention can be prepared in the form of powder through an additional process, such as vacuum distillation, lyophilizing or spray drying.

The *Magnolia sieboldii* extract of the present invention can exhibit the effect thereof by locally applying or spraying it on the skin. Thus, according to one embodiment of the present invention, the composition of the present invention can be prepared in the form of cosmetic compositions, including cream, lotion and skin lotion.

The content of the *Magnolia sieboldii* extract of the present invention is 0.0001-20.0 wt%, based on the total weight of the skin-whitening cosmetic composition. If the content of the *Magnolia sieboldii* extract is less than 0.00001 wt%, the effect thereof is difficult to show, and if it is more than 30 wt%, it has a high possibility of inducing skin irritation and can also have a great bad effect on the stabilization of a formulation. In a preferred embodiment of the present invention, a cosmetic composition comprising 2.0 wt% of the *Magnolia sieboldii* extract was prepared and applied directly on the skin, and as result, it was observed that it had an excellent skin-whitening effect.

Also, the skin-whitening cosmetic composition of the present invention comprises, in addition to the *Magnolia sieboldii* extract as an active ingredient, components which are conventionally used in cosmetic compositions, for example, conventional additives, such as an antioxidant, a stabilizer, a solubilizing agent, vitamins, a pigment and perfume, and carrier components.

The cosmetic composition of the present invention can be prepared in any formulation, which is conventionally prepared in the art. For example, it can be formulated into solution, suspension, emulsion, paste, gel, cream, lotion, powder, soap, surfactant-containing cleansing formulation, oil, powder foundation, emulsion foundation, wax foundation and spray, but the scope of the present invention is not limited thereto. More specifically, it can be prepared in the form of skin lotion, milk lotion, nourishing cream, massage cream, essence, eye cream, cleansing cream, cleansing foam, cleansing water, pack, spray or powder.

If the formulation of the present invention is paste, cream or gel, it may comprise, as carrier components, animal oil, vegetable oil, wax, paraffin, starch, tragacanth, cellulose derivatives, polyethylene glycol, silicone, bentonite, silica, talc or zinc oxide.

If the formulation of the present invention is powder or spray, it may comprise, as carrier components, lactose, talc, silica, aluminum hydroxide, calcium silicate or polyamide powder. Particularly, if it is spray, it may additionally comprise a propellant, such as chlorofluorohydrocarbon, propane/butane or dimethyl ether.

If the formulation of the present invention is solution or emulsion, it may comprise, as carrier components, a solvent, a solubilizing agent or an emulsifying agent, for example, water, ethanol, isopropanol, ethyl carbonate, ethyl acetate, benzyl alcohol, benzyl benzoate, propylene glycol, 1,3-butyl glycol oil, glycerol fatty ester, polyethylene glycol or sorbitan fatty acid ester.

If the formulation of the present invention is suspension, it may comprise, as carrier components, a liquid diluent, such as water, ethanol or propylene glycol, a suspending agent, such as ethoxylated isostearyl alcohol, polyoxyethylene sorbitol ester or polyoxyethylene sorbitan ester, microcrystalline cellulose, aluminum metahydroxide, bentonite, agar or tragacanth.

If the formulation of the present invention is a surfactant-containing cleansing formulation, it may comprise, as carrier components, aliphatic alcohol sulfate, aliphatic alcohol ether sulfate, sulfosuccinic monoester, isethionate, imidazolium derivatives, methyl taurate, sarcosinate, fatty acid amide ether sulfate, alkyl amido betaine, aliphatic alcohol, fatty acid glyceride, fatty acid diethanolamide, vegetable oil, lanoline derivatives, or ethoxylated glycerol fatty acid ester.

The cosmetic composition of the present invention can be used alone or in combination with other cosmetic compositions. Also, the inventive cosmetic composition having an excellent skin-protecting effect can be used according to any conventional method, and the number of use thereof can vary depending on the skin condition or liking of a user.

If the cosmetic composition of the present invention is soap, a surfactant-containing cleansing formulation or a cleansing formulation containing no surfactant, it can be wiped, removed or washed with water, after it is applied to the skin. Concrete examples of said soap include, but are not limited to, liquid soap, powder soap, solid soap and oil soap, examples of said surfactant-containing cleansing formulation include, but are not limited to, cleansing foam, cleansing water, cleansing towel and cleansing pack, and examples of said surfactant-not containing cleansing formulation include, but are not limited to, cleansing cream, cleansing lotion, cleansing water and cleansing gel.

Hereinafter, the present invention will be described in further detail with reference to examples. It is to be understood, however, that these examples are illustrative only, and the scope of the present invention is not limited thereto.

Preparation Example 1: Preparation I of *Magnolia sieboldii* extract

200 g of the flowers of *Magnolia sieboldii,* washed with purified water and dried, were added to 1.2 L of water and extracted by heating in an extractor, equipped with a cooling condenser, at 70-90 °C for 5 hours. Then, the extract was filtered through 300-mesh filter cloth, aged by allowing it to stand at 5-10 °C for 7-10 days, and then filtered through Whatman No. 5 filter paper. The filtrate was dried in a rotary vacuum evaporator at 65 °C, thus obtaining a dry weight of 10.8 g.

Preparation Example 2: Preparation II of _Magnolia sieboldii_ extract

200 g of the flowers of _Magnolia sieboldii,_ washed with purified water and dried, were added to 1.2 L of water, extracted at 15-35 °C for 5 days, filtered through 300-mesh filter cloth, and further filtered through Whatman No. 5 filter paper. Then, the filtrate was concentrated two-fold in a rotary vacuum evaporator. Then, 0.6 L of 100% ethanol was added to the concentrate, aged by allowing it to stand at 5-10 °C for 7-10 days, and then filtered through Whatman No. 5 filter paper. The filtrate was dried in a rotary vacuum evaporator at 65 °C, thus obtaining a dry weight of 12.5 g.

Preparation Example 3: Preparation III of _Magnolia sieboldii_ extract

200 g of the flowers of _Magnolia sieboldii,_ washed with purified water and dried, were added to 1.2 L of water, extracted at 4-40 °C for 5 days, filtered through 300-mesh filter cloth, aged by allowing it to stand at 5-10 °C for 7-10 days, and then filtered through Whatman No. 5 filter paper. The filtrate was dried in a rotary vacuum evaporator, thus obtaining a dry weight of 10.6 g.

Preparation Examples 4-21: Preparation IV of _Magnolia sieboldii_ extract

200 g of the flowers of _Magnolia sieboldii,_ washed with purified water and dried, were added to 1.2 L of each of the extraction solvents shown in Table 1 below and was extracted in the same manner as in Preparation Example 3. The extraction results are shown in Table 1.

[Table 1]

| Preparation Examples | Extraction solvents | Dry weight of final extract(unit : g) |
| --- | --- | --- |
| Preparation Example 4 | 10% ethanol | 12.9 |
| Preparation Example 5 | 20% ethanol | 13.3 |
| Preparation Example 6 | 30% ethanol | 14.2 |
| Preparation Example 7 | 40% ethanol | 13.5 |
| Preparation Example 8 | 50% ethanol | 13.6 |
| Preparation Example 9 | 60% ethanol | 14.7 |
| Preparation Example 10 | 70% ethanol | 15.8 |
| Preparation Example 11 | 80% ethanol | 14.5 |
| Preparation Example 12 | 90% ethanol | 13.6 |
| Preparation Example 13 | 100% ethanol | 12.1 |
| Preparation Example 14 | Methanol | 13.2 |
| Preparation Example 15 | n-propanol | 10.5 |
| Preparation Example 16 | Isopronanol | 10.6 |
| Preparation Example 17 | 2-butanol | 11.2 |
| Preparation Example 18 | Acetone | 10.2 |
| Preparation Example 19 | Chloroform | 10.5 |
| Preparation Example 20 | Ethvl acetate | 10.6 |
| Preparation Example 21 | Butyl acetate | 10.3 |

Preparation Example 22: Preparation V of _Magnolia sieboldii_ extract

200 g of the flowers of _Magnolia sieboldii,_ washed with purified water and dried, were added to 1.2 L of 1,3-butylene glycol, extracted for 48 hours, filtered through 300-mesh filter cloth, aged by allowing it at 5-10 °C for 7-10 days, and then filtered through Whatman No. 5 filter paper. The extract was subjected to the loss of dry weight, thus obtaining a final concentration of 1% (w/v).

Preparation Example 23: Preparation VI of *Magnolia sieboldii* extract

200 g of the flowers of *Magnolia sieboldii,* washed with purified water and dried, were added to 1.2 L of 10% ethanol and extracted by heating in an extractor equipped with a cooling condenser for 5 hours. Then, the extract was filtered through 300-mesh filter cloth, aged by allowing it to stand at 5-10 °C for 7-10 days, and then filtered through Whatman No. 5 filter paper. The extract was dried in a rotary vacuum evaporator at 65 °C, thus obtaining a dry weight of 13.8 g.

Preparation Example 24: Preparation VII of *Magnolia sieboldii* extract

200 g of the flowers of *Magnolia sieboldii,* washed with purified water and dried, were added to 1.2 L of each of the extraction solvents shown in Table 2 below and was extracted in the same manner as in Example 23. The extraction results are shown in Table 2.

[Table 2]

| Preparation Examples | Extraction solvents | Dry weight of final extract (unit : g) |
|---|---|---|
| Preparation Example 24 | 20% ethanol | 13.8 |
| Preparation Example 25 | 30% ethanol | 13.6 |
| Preparation Example 26 | 40% ethanol | 14.9 |
| Preparation Example 27 | 50% ethanol | 13.6 |
| Preparation Example 28 | 60% ethanol | 14.5 |
| Preparation Example 29 | 70% ethanol | 16.3 |
| Preparation Example 30 | 80% ethanol | 13.5 |
| Preparation Example 31 | 90% ethanol | 14.6 |
| Preparation Example 32 | 100% ethanol | 13.8 |

Test Example 1: Tyrosinase activity inhibitory effect of *Magnolia* sieboldii extract

40 $\mu\ell$ of 1.5 mmol/l tyrosine (Sigma, USA) in sodium phosphate buffer (pH 6.8) was used as a substrate, and each of the *Magnolia sieboldii* extracts prepared in Preparation Examples 1-32 was diluted in 0.05 M sodium phosphate buffer (pH 6.8), thus preparing sample solutions. 240 $\mu\ell$ of each of the sample solutions was added to 40 $\mu\ell$ of tyrosine, 20 $\mu\ell$ of tyrosinase (1500 U/ m$\ell$; Sigma) was added to the solution. Then, the mixture solution was allowed to react at 37°C for 15 minutes, and then measured for absorbance at 490 nm. Herein, a solution having no *Magnolia sieboldii* extract added thereto was used as a control group. The inhibitory rate of tyrosinase activity was calculated according to the following equation 1, and the calculation results are shown in Table 3 below:

【Math Figure 1】

$$\text{Tyrosinase activity inhibitory rate (\%)} = \{1-\text{absorbance (O.D.490) of comparative group/absorbance (O.D.490) of control group}\} \times 100$$

[Table 3]

| Tyrosinase activity inhibitory effect of *Magnolia sieboldii* extract (test concentration: 100 $\mu$g/m$\ell$) | | | |
|---|---|---|---|
| Test substances | Tyrosinase activity inhibitory rate (%) | Test substances | Tyrosinase activity inhibitory rate (%) |
| Preparation Example 1 | 45.8 | Preparation Example 17 | 52.5 |

(continued)

| Tyrosinase activity inhibitory effect of *Magnolia sieboldii* extract (test concentration: 100 μg/mℓ) | | | |
|---|---|---|---|
| Test substances | Tyrosinase activity inhibitory rate (%) | Test substances | Tyrosinase activity inhibitory rate (%) |
| Preparation Example 2 | 50.6 | Preparation Example 18 | 56.3 |
| Preparation Example 3 | 52.6 | Preparation Example 19 | 54.5 |
| Preparation Example 4 | 54.2 | Preparation Example 20 | 58.2 |
| Preparation Example 5 | 55.8 | Preparation Example 21 | 53.2 |
| Preparation Example 6 | 50.3 | Preparation Example 22 | 54.2 |
| Preparation Example 7 | 48.5 | Preparation Example 23 | 50.2 |
| Preparation Example 8 | 49.6 | Preparation Example 24 | 55.6 |
| Preparation Example 9 | 50.2 | Preparation Example 25 | 52.3 |
| Preparation Example 10 | 51.3 | Preparation Example 26 | 50.3 |
| Preparation Example 11 | 52.2 | Preparation Example 27 | 55.6 |
| Preparation Example 12 | 50.3 | Preparation Example 28 | 54.3 |
| Preparation Example 13 | 52.5 | Preparation Example 29 | 58.5 |
| Preparation Example 14 | 56.3 | Preparation Example 30 | 58.8 |
| Preparation Example 15 | 58.5 | Preparation Example 31 | 59.9 |
| Preparation Example 16 | 51.2 | Preparation Example 32 | 60.3 |

As can be seen from the results in Table 3 above, the *Magnolia sieboldii* extract samples, obtained through various extraction methods, showed tyrosinase activity inhibitory effects. This suggests that the inventive composition comprising the *Magnolia sieboldie* extract shows an excellent effect of inhibiting tyrosinase activity.

Test Example 2: Melanin production inhibitory effect of *Magnolia* sieboldii extract (melanin analysis)

B-16 cells (mouse melanoma cells, Korea Cell Line Bank) were inoculated onto a 12- well plate at a density of 10 cells/well, and then cultured for one day. Each well was treated with various concentrations of the *Magnolia sieboldii* extract and cultured for 3-4 days, and the culture medium in each well was centrifuged. The isolated cells were lysed with a solution of 500 μℓ of 1N NaOH in dimethylsulfoxide (DMSO) and heated in a water bath at 90 °C for 10 minutes. The lysed cells were centrifuged again, and the absorbance of the supernatant at 570 nm was measured. As a control group, cells untreated with the *Magnolia sieboldii* extract were used. The inhibitory rate of melanin production was calculated according to the following equation 2, and the calculation results are shown in Table 4 below:

【Math Figure 2】

$$\text{Melanin production inhibitory rate (\%) = \{1-absorbance (O.D.490) of comparative group/absorbance (O.D.490) of control group\} x 100}$$

[Table 4]

| Test substances | Melanin production inhibitory rate (%) | Test substances | Melanin production inhibitory rate (%) |
| --- | --- | --- | --- |
| Preparation Example 1 | 66.8 | Preparation Example 17 | 63.6 |
| Preparation Example 2 | 65.7 | Preparation Example 18 | 65.2 |
| Preparation Example 3 | 65.3 | Preparation Example 19 | 68.3 |
| Preparation Example 4 | 64.3 | Preparation Example 20 | 67.5 |
| Preparation Example 5 | 66.7 | Preparation Example 21 | 65.3 |
| Preparation Example 6 | 67.3 | Preparation Example 22 | 65.3 |
| Preparation Example 7 | 68.6 | Preparation Example 23 | 66.3 |
| Preparation Example 8 | 66.7 | Preparation Example 24 | 65.5 |
| Preparation Example 9 | 65.3 | Preparation Example 25 | 63.3 |
| Preparation Example 10 | 66.3 | Preparation Example 26 | 65.2 |
| Preparation Example 11 | 63.2 | Preparation Example 27 | 64.2 |
| Preparation Example 12 | 65.3 | Preparation Example 28 | 68.3 |
| Preparation Example 13 | 63.4 | Preparation Example 29 | 68.5 |
| Preparation Example 14 | 65.3 | Preparation Example 30 | 68.7 |
| Preparation Example 15 | 68.5 | Preparation Example 31 | 69.8 |
| Preparation Example 16 | 63.2 | Preparation Example 32 | 67.3 |

As can be seen from the results in Table 4 above, it was confirmed that the *Magnolia sieboldii* extract had an excellent effect of inhibiting the melanin production of melanoma cells. Also, in each of the tests, no cytotoxicity was observed.

Hereinafter, formulation examples of the inventive cosmetic composition comprising the *Magnolia sieboldii* extract as an active ingredient will be described in detail, but the inventive cosmetic composition is not limited to these formulation examples. Also, the *Magnolia sieboldii* extract used in the following formulation examples was a material obtained by dissolving the material of Preparation Example 10 in 30% 1,3-butylene glycol at a concentration of 1% (w/v).

Formulation Example 1: Skin lotion

Among cosmetic compositions comprising the *Magnolia sieboldii* extract, a skin lotion formulation is shown in Table 5 below.

[Table 5]

| Components | Contents (wt%) |
|---|---|
| *Magnolia sieboldii* extract | 2.0 |
| Glycerin | 5.0 |
| 1.3-butylene glycol | 3.0 |
| PEG 1500 | 1.0 |
| Allantoin | 0.1 |
| DL-pantenol | 0.3 |
| EDTA-2NA | 0.02 |
| Benzophenone-9 | 0.04 |
| Sodium hvaluronate | 5.0 |
| Ethanol | 10.0 |
| Ortvl dodeses-16 | 0.2 |
| Polvsorbate 20 | 0.2 |
| Preservative, perfume and pigment | q.s. |
| Distilled water | balance |
| Total | 100 |

Formulation Example 2: Astringent lotion

Among cosmetic compositions comprising the *Magnolia sieboldii* extract, an astringent lotion formulation is shown in Table 6 below.

[Table 6]

| Components | Contents (wt%) |
|---|---|
| *Magnolia sieboldii* extract | 2.0 |
| Glycerin | 2.0 |
| 1.3-butvlene glycol | 2.0 |
| Allantoin | 0.2 |
| DL-pantenol | 0.2 |
| EDTA-2Na | 0.02 |
| Benzophenone -9 | 0.04 |
| Sodium hyaluronate | 3.0 |
| Ethanol | 15.0 |
| Polvsorbate 20 | 0.3 |
| Witch Hazel extract | 2.0 |

(continued)

| Components | Contents (wt%) |
|---|---|
| Citric acid | q.s |
| Preservative, perfume and pigment | q.s |
| Distilled water | balance |
| Total | 100 |

Formulation Example 3: Milk lotion

Among cosmetic compositions comprising the *Magnolia sieboldii* extract, a milk lotion formulation is shown in Table 7 below.

[Table 7]

| Components | Contents (wt%) |
|---|---|
| *Magnolia sieboldii* extract | 2.0 |
| Glyceryl stearate SE | 1.5 |
| Stearvl alcohol | 1.5 |
| Lanolin | 1.5 |
| Polvsorbate 60 | 1.3 |
| Sorbitan stearate | 0.5 |
| Hvdrogenated vegetable oil | 1.0 |
| Mineral oil | 5.0 |
| Squalane | 3.0 |
| Tiroctanoin | 2.0 |
| Dimethicone | 0.8 |
| Carboxyvinvl polymer | 0.5 |
| Glycerin | 0.12 |
| 1.3-butvlenealvcol | 5.0 |
| Sodium hvaluronate | 3.0 |
| Triethanolamine | 0.12 |
| Preservative, perfume and pigment | q.s |
| Distilled water | balance |
| Total | 100 |

Formulation Example 4: Nourishing cream

Among cosmetic compositions comprising the *Magnolia sieboldii* extract, a nourishing cream formulation is shown in Table 8 below.

[Table 8]

| Components (content: wt%) | Example 1 | Comparative Example 1 |
|---|---|---|
| *Magnolia sieboldii* extract | 2.0 | - |
| Lipophilic glycerin monostearate | 2.0 | 2.0 |

(continued)

| Components (content: wt%) | Example 1 | Comparative Example 1 |
|---|---|---|
| Cetearyl alcohol | 2.2 | 2.2 |
| Stearic acid | 1.5 | 1.5 |
| Beewax | 1.0 | 1.0 |
| Polysorbate 60 | 1.5 | 1.5 |
| Sorbitan stearate | 0.6 | 0.6 |
| Hydrogenated vegetable oil | 1.0 | 1.0 |
| Squalane | 3.0 | 3.0 |
| Mineral oil | 5.0 | 5.0 |
| Trioctanoin | 5.0 | 5.0 |
| Dimethicone | 1.0 | 1.0 |
| Sodium magnesium silicate | 0.1 | 0.1 |
| Glycerin | 5.0 | 5.0 |
| Betaine | 3.0 | 3.0 |
| Triethanolamine | 1.0 | 1.0 |
| Sodium hvaluronate | 4.0 | 4.0 |
| Preservative, perfume and pigment | q.s. | q.s. |
| Distilled water | balance | balance |
| Total | 100 | 100 |

Formulation Example 5: Massage cream

Among cosmetic compositions comprising the *Magnolia sieboldii* extract, a massage cream formulation is shown in Table 9 below.

[Table 9]

| Components | Contents (wt%) |
|---|---|
| *Magnolia sieboldii* extract | 2.0 |
| Lipophilic glycerin monostearate | 1.5 |
| Stearvl alcohol | 1.5 |
| Stearic acid | 1.0 |
| Polvsorbate 60 | 1.5 |
| Sorbitan stearate | 0.6 |
| Isostearyl isostearate | 5.0 |
| Squalane | 5.0 |
| Mineral oil | 35.0 |
| Dimethicone | 0.5 |
| Hvdroxvethylcellulose | 0.12 |
| Glycerin | 6.0 |
| Triethanolamine | 0.7 |
| Preservative, perfume and pigment | q.s |

(continued)

| Components | Contents (wt%) |
|---|---|
| Distilled water | balance |
| Total | 100 |

Formulation Example 6: Essence

Among cosmetic compositions comprising the *Magnolia sieboldii* extract, an essence formulation is shown in Table 10 below.

[Table 10]

| Components | Contents (wt%) |
|---|---|
| *Magnolia sieboldii* extract | 2.0 |
| Glycerin | 10.0 |
| Betaine | 5.0 |
| PEG 1500 | 2.0 |
| Allantoin | 0.1 |
| DL-pantenol | 0.3 |
| EDTA-2Na | 0.02 |
| Benzophenone - 9 | 0.04 |
| Hydroxyethylcellulose | 0.1 |
| Sodium hyaluronate | 8.0 |
| Carboxyvinyl polymer | 0.2 |
| Triethanolamine | 0.18 |
| Octyl dodecanol | 0.3 |
| Octyl dodeses-16 | 0.4 |
| Ethanol | 6.0 |
| Preservative, perfume and pigment | q.s. |
| Distilled water | balance |
| Total | 100 |

Formulation Example 7: Pack

Among cosmetic compositions comprising the *Magnolia sieboldii* extract, a pack formulation is shown in Table 11 below.

[Table 11]

| Component | Contents (wt%) |
|---|---|
| *Magnolia sieboldii* extract | 2.0 |
| Polyvinyl alcohol | 15.0 |
| Cellulose gum | 0.15 |
| Glycerin | 3.0 |
| PEG 1500 | 2.0 |

(continued)

| Component | Contents (wt%) |
|---|---|
| Cyclodextrin | 0.15 |
| DL - pantenol | 0.4 |
| Allantoin | 0.1 |
| Ammonium glycyrrhizinate | 0.3 |
| Nicotinamide | 0.5 |
| Ethanol | 6.0 |
| PEG 40, hydrogenated castor oil | 0.3 |
| Preservative, perfume and pigment | q.s. |
| Distilled water | balance |
| Total | 100 |

Test Example 4: Test of whitening effect of cosmetic composition comprising *Magnolia sieboldii* extract

A test of the whitening effect of the inventive cosmetic composition comprising the *Magnolia sieboldii* extract was carried out on twenty 19-40-year-old Korean women having a black skin. As measurement sites, three sites, each having an area of 1 $cm^2$, were marked on the arm of each of the test subjects. The two sites were applied with a cosmetic composition of Example 1, comprising the *Magnolia sieboldii* extract and prepared according to the method of Formulation Example 4, and a cosmetic composition of Comparative Example 1, not comprising the *Magnolia sieboldii* extract, respectively, and the remaining one site was used as a control group for comparison. Each of the cosmetic compositions was continuously applied for 8 weeks at an interval of 2 weeks, and after 8 weeks, the skin color (L value) was measured using Minolta CR 300. Then, the change in skin color (ΔL) was calculated according to Equation 3, and the calculation results are shown in Table 12 below.

[Table 12]

| | Example 1 | Comparative Example 1 |
|---|---|---|
| ΔL value | 1.60 | 0.50 |

As can be seen in Table 12 which shows the change in skin color (ΔL) after 8 weeks, the cosmetic formulation of Example 1, comprising the *Magnolia sieboldii* extract, showed a high whitening effect. This suggests that the inventive cosmetic composition comprising the *Magnolia sieboldii* extract has a very excellent whitening effect.

**Claims**

1. Use of a cosmetic composition for skin-whitening comprising an extract of *Magnolia sieboldii* as an active ingredient, wherein the *Magnolia sieboldii* extract is comprised in an amount of 0.0001-20.0 wt% based on the total weight of the cosmetic composition.

2. The use of a cosmetic composition for skin-whitening as defined in Claim 1, wherein the *Magnolia sieboldii* extract is obtained by extraction with an extraction solvent selected from the group consisting of water, an anhydrous or hydrous lower alcohol having 1-4 carbon atoms, acetone, ethyl acetate and butyl acetate.

3. The use of a cosmetic composition for skin-whitening as defined in Claim 1 or 2, wherein the cosmetic composition is a formulation selected from the group consisting of solution, suspension, emulsion, paste, gel, cream, lotion, powder, soap, surfactant-comprising cleansing formulation, oil, powder foundation, emulsion foundation, wax foundation and spray.

**Patentansprüche**

1.  Verwendung einer Kosmetikzusammensetzung, die einen Extrakt von *Magnolia sieboldii* als Wirkstoff umfasst, zur Hautaufhellung, wobei der *Magnolia-sieboldii*-Extrakt in einer Menge von 0,0001 bis 20,0 Gew.-% vorhanden ist, bezogen auf das Gesamtgewicht der Kosmetikzusammensetzung.

2.  Verwendung einer Kosmetikzusammensetzung zur Hautaufhellung gemäß Anspruch 1, wobei der *Magnolia-sieboldii*-Extrakt durch Extraktion mit einem Extraktionslösungsmittel, das aus der Gruppe ausgewählt ist, die aus Wasser, einem wasserfreien oder wässrigen niederen Alkohol mit 1 bis 4 Kohlenstoffatomen, Aceton, Ethylacetat und Butylacetat besteht, erhalten wird.

3.  Verwendung einer Kosmetikzusammensetzung zur Hautaufhellung gemäß Anspruch 1 oder 2, wobei die Kosmetikzusammensetzung eine Zubereitung ist, die aus der Gruppe ausgewählt ist, die aus einer Lösung, Suspension, Emulsion, Paste, einem Gel, einer Creme, Lotion, einem Pulver, einer Seife, tensidhaltigen Reinigerzubereitung, einem Öl, einer Puder-Foundation, einer Flüssig-Foundation, einer Wachs-Foundation und einem Spray besteht.


**Revendications**

1.  Utilisation d'une composition cosmétique pour blanchir la peau, comprenant un extrait de *Magnolia sieboldii* en tant qu'ingrédient actif, l'extrait de *Magnolia sieboldii* étant présent en une quantité de 0,0001-20,0 % en poids par rapport au poids total de la composition cosmétique.

2.  Utilisation d'une composition cosmétique pour blanchir la peau telle que définie dans la revendication 1, où l'extrait de *Magnolia sieboldii* est obtenu par extraction à l'aide d'un solvant d'extraction choisi dans le groupe constitué par l'eau, un alcool inférieur anhydre ou hydraté ayant 1-4 atomes de carbone, l'acétone, l'acétate d'éthyle et l'acétate de butyle.

3.  Utilisation d'une composition cosmétique pour blanchir la peau telle que définie dans la revendication 1 ou 2, ladite composition cosmétique étant une formulation choisie dans le groupe constitué par une solution, une suspension, une émulsion, une pâte, un gel, une crème, une lotion, une poudre, un savon, une formulation nettoyante comportant un agent tensioactif, une huile, un fond de teint poudre, un fond de teint émulsion, un fond de teint cire et un spray.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI49320 B
- JP HEI6192062 B
- JP SHO567710 B
- JP HEI49315 B